# EUROPEAN PATENT APPLICATION

(11) **EP 0 582 441 A1**
(43) Date of publication of application: **09.02.1994**
(21) Application number: 93306010.5
(22) Date of filing: 29.07.1993
(51) Int. Cl.: C07C 233/73

(54) **Processes for the preparation of tyramine derivatives**

(30) Priority: 02.08.1992 IL 102707
(71) Applicant: CHEMAGIS LTD., Tel Aviv 61090 (IL)
(72) Inventor: Cherkez, Stephen, Ramat Gan (IL); Yigal, Deborah, Kfar Shmariyahu (IL); Segal,Ariana, Tel Aviv (IL); Rona, Peter, Haifa (IL); Feinberg, Jan, Tel Aviv (IL)
(74) Representative: Ablewhite, Alan James

(57) **Abstract**

The invention provides a process for the production of N-[2-(p-hydroxyphenyl)ethyl]-p-chlorobenzamide of the formula III: 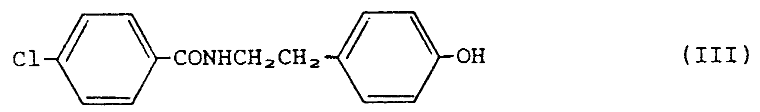
and derivatives thereof, comprising reacting 4-(2-aminoethyl) phenol (Tyramine) of formula I: 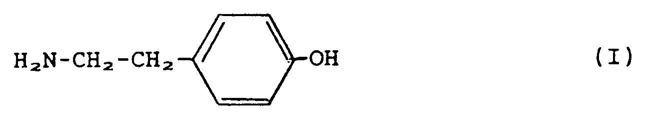
or the hydrochloride salt thereof, with 1.0 to 1.5 molar equivalent of 4-chlorobenzoyl chloride in the presence of water, in the presence of at least one molar equivalent of base and in the presence of an organic solvent.

## Description

The present invention relates to a process for the preparation of derivatives of 4-(2-aminoethyl)phenol (Tyramine) of formula I:

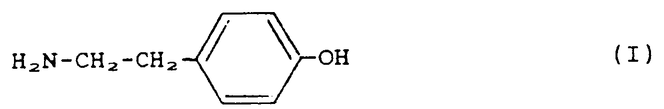

More particularly the present invention relates to a process for the production of N-[2-(p-hydroxphenyl)ethyl]-p-chloro-benzamide of the formula III: 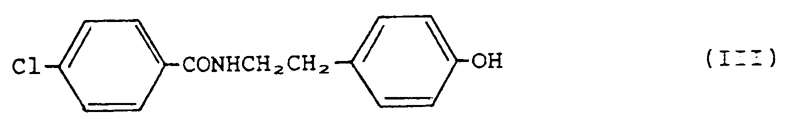
and derivatives thereof.

Tyramine is a bifunctional compound possessing an amine and a phenol function. Both functional groups are reactive. Being an amine, Tyramine forms salts with acids.

The process of the present invention relates to reactions of Tyramine (free base) which may be obtained and used as such, or may be liberated from its salts in the course of the process by the addition of a predetermined quantity of a suitable reagent. Due to the reactive nature of the functional groups which are a constituent part of the molecule, by attaching various groups to the amine and phenol moieties, Tyramine may easily be derivatized. Derivatization can take place non-selectively, yielding mono- di- and/or unsubstituted molecules. A second derivatization complicates even more this situation. This situation poses a disadvantage especially when only one group needs to be derivatized at a time.

The object of the present invention is to provide a process for the selective sequential reaction of Tyramine whereby the two reactive functional groups of the molecule react with different reagents in a manner such that reaction with the first reagent will take place selectively only on the desired one of the two reactive functions, leaving the second function open for reaction with the second reagent in a subsequent stage.

It is also an object of the present invention to show that the present process in addition to being sequentially selective will, if so desired, also be applicable in a continuous manner. Namely, that the product A, formed in the course of the process by the reaction of Tyramine with the first reagent, can be isolated, or, if so desired, the process may be continued by reacting the product (A) without isolation with the second reagent to produce product B according to the following reaction sequence:

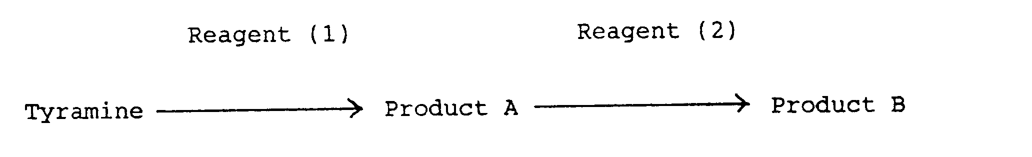

An additional object of this invention is to provide means to allow for the facile purification of the products of the said process either when isolated in the said manner after reacting with the first reagent or when reacted in the said manner continuously without isolation with the second reagent. The process which is the object of this invention provides improved yields as well as improved methods of reaction, work-up, isolation and purification of the desired selected derivatives of Tyramine.

An important selectively functionalized derivative of Tyramine is 2-[4-[2-[(4-chlorobenzoyl)amino]ethyl]phenoxy]-2-methyl propanoic acid of the formula II:

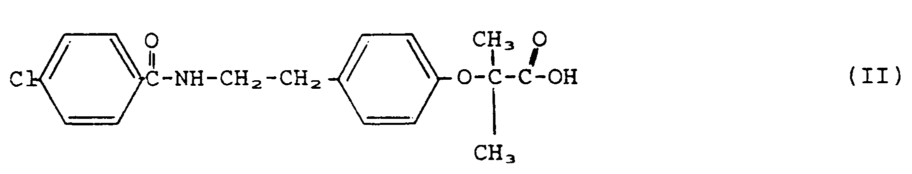

This derivative is a highly active therapeutic material used in pharmaceutical preparations as an antihyperlipoproteinemic agent. The general approach to the preparation of II, as described, inter alia, in the following references:
1. E. C. Witte, K. Stack, M. Thiel and H. Stork, Ger. Pat. 2,149,070 (1977);
2. P. Beyer, Brit. Pat. 2,021,575 (1979);
3. P. Beyer, Ger. Pat. 2,920,413 (1979);
4. D. F. Molina Caprile, Span. Pat. 505777 (1981); and
5. J. P. Bourgogne and R. Somsy, European Patent No. 245,156 (1987);

is based on the isolation of N-[2-(p-hydroxphenyl)ethyl]-p-chlorobenzamide of the formula III: 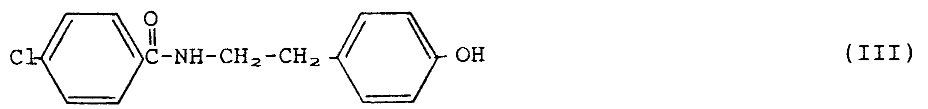
from the reaction mixture usually after an intermediate step.
The production of III, due to the absence of selectivity in the specified processes (Refs. 1-3) usually takes place through the intermediacy of an intermediate of the general formula IV: 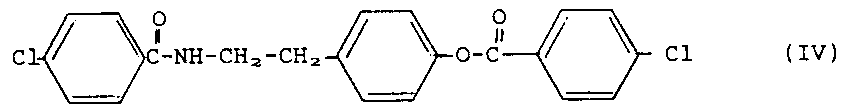
The production of IV is disadvantageous for numerous reasons since:
a. double the amount of a reagent (4-chlorobenzoyl chloride) is necessary than the quantity actually needed for the formation of III;
b. to obtain III, the initially formed compound, IV, must be selectively hydrolysed;
c. in order to allow for the further reaction of III it must be freed from the by-product of the hydrolysis;
d. in order to utilize the recovered by-product, it must be recycled.

Production of this key intermediate is specified also in Spanish Patent No. 505777 (Ref. 4).

In the process specified in Ref. 4, compound III is produced by reaction of one equivalent of 4-chlorobenzoyl chloride with two equivalents of Tyramine. The product is a mixture of III and Tyramine hydrochloride. Tyramine hydrochloride is separated and recycled, while III is isolated and purified. This approach to III suffers, inter alia, from the disadvantages of using an expensive reactant (Tyramine) as a neutralization reagent.

With respect to the one-pot derivatization of Tyramine in the course of the production of II from III it is especially noteworthy that in all the published procedures (Refs. 1-5), isolated III is employed which is also prepurified in some way as a means to eliminate admixed by-product.

The present invention provides a novel process for the preparation of certain Tyramine derivatives of the formula II and its precursor [2-(p-hydroxphenyl)ethyl]-p-chlorobenzamide of the formula III. As indicated hereinbefore, the production of II according to the prior art (Refs. 1-3), is effected through the reaction of its isolated precursor III. While to obtain III, according to the prior art an additional intermediate, IV, must be produced.

Suprisingly and unexpectedly, in the framework of the present invention it was found that, under the conditions specified hereinafter, the formation of the unwanted di-4-chlorobenzoyl intermediate, IV, is avoided. This pathway provides directly the desired material, III, which may be employed in the next synthetic step with or without isolation and/or further purification. Thus according to the present invention there is now provided a process for the production of N-[2-(p-hydroxyphenyl)ethyl]-p-chloro-benzamide of the formula III: 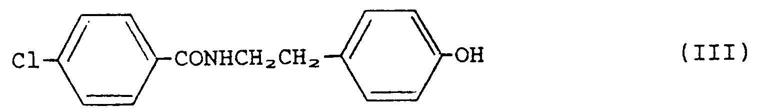
and derivatives thereof comprising reacting 4-(2-aminoethyl) phenol (Tyramine) of formula I: 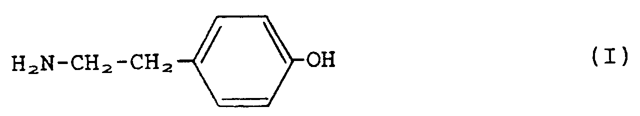
or the hydrochloride salt thereof with 1.0 to 1.5 molar equivalent of 4-chlorobenzoyl chloride in the presence of water, in the presence of at least one molar equivalent of base and in the presence of an organic solvent.

The invention also provides a unitary process for the production of the 2-[4-[2-[(4-chlorobenzoyl)amino]ethyl]phenoxy]-2-methyl propanoic acid derivative of N-[2-(p-hydroxyphenyl)-ethyl]-p-chlorobenzamide in situ comprising reacting Tyramine hydrochloride with 1.0 to 1.35 molar equivalent of 4-chlorobenzoyl chloride in the presence of:
a. water;
b. at least one molar equivalent of sodium or potassium hydroxide;
c. at least one molar equivalent of a weak base selected from sodium bicarbonate, potassium bicarbonate, sodium carbonate, potassium carbonate or pyridine and trialkylamine of the formula NR₃ wherein R is CH₃, C₂H₅, C₃H₇, C₄H₉, C₅H₁₁, or a mixture thereof;
d. a lower aliphatic ester of 2-bromoisobutyric acid, a lower aliphatic ester of 2-chloroisobutyric acid or a mixture thereof; and
e. a high boiling hydroxylic solvent to produce isopropyl 2-[4-[2(4-chlorobenzoyl)amino]ethyl]phenoxy]-2-methyl propanoate of the general formula V: 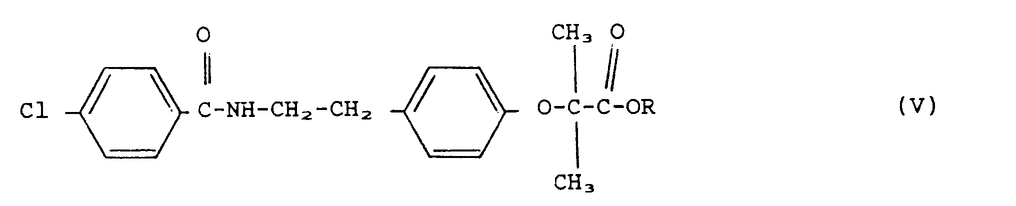 which ester is then hydrolyzed to form II through reaction with an aqueous solution of potassium hydroxide or sodium hydroxide or mixtures thereof in the temperature range of 40-90°C, preferably 65-75°C.

The direct and selective formation of III is effected by the reaction of Tyramine hydrochloride in the aqueous medium in the presence of about 1 molar equivalent of base as described hereinafter. In the event that Tyramine hydrochloride is used, an additional equivalent of aqueous sodium or potassium hydroxide solution or any other suitable base is added to the mixture to liberate the Tyramine free base from its hydrochloride. Into this mixture one or more additional organic solvents, said solvent being preferably an ester, ketone or ether, are added, at a volume per weight ratio of about 0.3 to 10, preferably 1 to 5 volumes per weight of Tyramine or Tyramine hydrochloride. The mixture is treated with an equivalent or a moderate excess of 4-chlorobenzoyl chloride in the presence of an equivalent or a moderate excess of sodium or potassium bicarbonate or carbonate or a weak base such as pyridine.

In the framework of the present invention it is expressedly specified that a lower ester of 2-bromo or 2-chloro isobutyric acid may also be used as the "additional organic component" (solvent).

Esters of this particular acid are an especially preferred component, since in a later stage of the process these serve as the reactant necessary for completion of the derivatization of Tyramine through the transformation of III into a lower aliphatic ester of 2-[4-[2(4-chlorobenzoyl)amino]ethyl]phenoxy]-2-methyl propanoic acid of the general formula V: 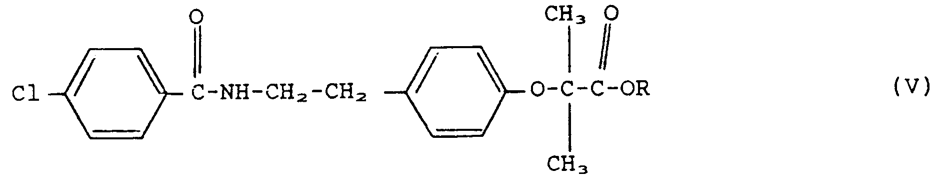
wherein R is methyl, ethyl, n-propyl or isopropyl.

The reaction is performed
a. through the gradual addition of 4-chlorobenzoyl chloride to the stirred mixture, in the temperature range of 20-80°C, preferably 40-60°C at this stage, subject to the choice of the "additional organic component" (solvent), the intermediate product III may be isolated and transformed in a separate reaction (v.i.) into the ester V; or
b. given that the "additional organic component" (solvent) is a lower aliphatic ester of 2-bromo or 2-chloroisobutyric acid, the reaction sequence may be continued to the ester V without the isolation and/or purification of intermediate III.

An additional alternative is that a mixture of "additional organic components" is employed in the reaction. When proceeding without isolation of III, that is when the "additional organic component" (solvent) is an ester of 2-bromo or 2-chloro-isobutyric acid, the mixture is heated to reflux for a period of 1-3 hours, one equivalent of sodium (or potassium) hydroxide and a high boiling hydroxylic solvent is added. The preferred hydroxylic solvent is an isomer or a mixture of isomers of octanol but other C₄ to C₁₂ aliphatic liquid primary or secondary alcohols may also be used.

The mixture is heated to reflux (100-110°C) for 0.5 to 5.0 hours, preferably 0.5 to 3.5 hours. In the course of the reflux, 40 to 60% of the intermediate, III, produced in the previous stage of the process, is converted to a lower aliphatic ester, of 2-[4-[2(4-chlorobenzoyl)amino]ethyl]phenoxy]-2-methyl propanoic acid of the formula V. At this stage the aqueous phase of the mixture is separated and discarded and to the organic phase one to six, preferably two to four, molar equivalents of dry potassium carbonate are added. The mixture is heated at a temperature of 115 to 165°C, preferably at 125 to 135°C, until conversion is complete.

The product is washed free of salts by the addition of water and separation of the aqueous phase. To the organic phase an aqueous solution of 20 to 70% weight, preferably 40 to 50% weight, potassium hydroxyde solution is gradually added. The mixture is heated to 45 to 90°C, preferably 55 to 65°C.

Following the completion of the hydrolysis, the mixture is diluted with water, and is allowed to separate to an organic phase which consists mostly of the C₄ - C₁₂ aliphatic alcohol and an aqueous layer. To the aqueous phase an organic solvent is added and the mixture is acidified to pH 3-4 at 60-80°C. The acid precipitates and is separated by centrifugation. Yield is 75-95% of the theoretical value.

In the framework of the process which is the subject of this invention, it was established that heating the crude product in an organic solvent in the presence of water when the combined volume of the organic solvent and the water applied is 1 to 10, preferably 2.5 to 5.0, times that of the dry weight of the crude product used, and in any particular case less than that necessary to fully dissolve the product, will nevertheless be sufficient to purify the acid to an extent comparable to that achieved by usual recrystallization e.g. by total dissolution of the material to be purified to achieve a homogeneous solution. Notwithstanding the above, in the case of a dry or aqueous solvent or solvents so specified, recrystallization by way of total dissolution through homogeneous aqueous or non-aqueous solutions may from time to time be the method of choice.

Thus the surprising and unexpected finding, that the extractive purification and recrystallization of the product using aqueous methyl isobutyl ketone as solvent constitutes an especially preferred embodiment of the present invention.
invention.

**TABLE 1**

| RECRYSTALLIZATION OF III | | | |
|---|---|---|---|
| Solvent | Nᵣ of Volumes per Weight of III | | Pure Product Yield. % |
| | Solvent | H₂O | |
| MIBK¹ | 5.5 | 0.3 | 93 |
| EtOAc² | 22.0 | 0.3 | 76 |
| MEK³ | 3.5 | 0.3 | 78 |

**TABLE 2**

| EXTRACTIVE PURIFICATION AND RECRYSTALLIZATION OF II | | | | | | | |
|---|---|---|---|---|---|---|---|
| Crude | III | Solvent | nₒ of Volumes per Weight III | | Pure II | | III |
| CBA¹ % | % | | Solvent | H₂O | CBA % | III % | Yield % |
| 3.5 | 0.52 | MIBK¹ | 5.0 | 0.6 | 0.19 | 0.12 | 79 |
| 3.0 | 1.04 | MIBK¹ | 5.0 | 0.5 | 0.02 | 0.30 | 84 |
| 3.0 | 1.04 | MIBK¹ | 7.7 | 0.5 | 0.07 | 0.34 | 71 |
| 3.0 | 0.25 | MIBK¹ | 4.2 | 0.3 | 0.02 | 0.15 | 84 |
| 3.0 | 1.04 | MEK³ | 4.0 | 0.55 | 0.08 | 0.26 | 56 |
| 2.7 | 0.40 | Acet:MeOH⁸ | 4.0 | 0.6 | 0.14 | 0.16 | 71 |
| 3.0 | 1.04 | IPA | 4.7 | 0.55 | 0.04 | 0.34 | 80 |

### Notes to Tables 1 and 2

1. Methyl isobutyl ketone
2. Ethyl acetate
3. Methyl ethyl ketone
4. 4-Chlorobenzoic acid
5. Acetone/Methanol 4 to 1 Vol/Vol.

While the methods of this invention will now be described in connection with certain preferred embodiments in the following examples, so that aspects thereof may be more fully understood and appreciated, it is not intended to limit the invention to these particular embodiments. On the contrary, it is intended to cover all alternatives, modifications and equivalents as may be included within the scope of the invention as defined by the appended claims. Thus, the following examples which include preferred embodiments will serve to illustrate the practice of this invention, it being understood that the particulars shown are by way of example and for purposes of illustrative discussion of preferred embodiments of the present invention only and are presented in the cause of providing what is believed to be the most useful and readily understood description of formulation procedures as well as of the principles and conceptual aspects of the invention.

### Example 1

### Preparation of N-[2-(p-hydroxyphenyl)ethyl]-p-chlorobenzamide, III

### Method A

In a 0.5 liter three-necked flask were placed 121 ml water, 43.4 g Tyramine hydrochloride, 23.1 g of a 46% by weight solution of sodium hydroxide, 24.4 g of sodium bicarbonate and 86 ml isopropyl acetate. The mixture was stirred and heated to 50°C and 44.3 g 4-chlorobenzoyl chloride was added dropwise. The mixture was cooled, the product was filtered off and washed with water. The dry product weighed 66.9 g, mp. 173.2-174.2°C. Yield: 97.2% III.

### Method B

The preparation was performed exactly as given in Method A while replacing isopropyl acetate with an identical volume of ethyl acetate. Weight of the product III obtained - 67.0 g; mp. 172-173°C. Yield: 95.7%.

### Method C

In a 2.0 liter flask were placed 620 ml water, 104.1 g Tyramine hydrochloride and 150 g sodium bicarbonate. To this stirred suspension 310 ml acetone was gradually added with cooling. To this mixture was added dropwise with cooling over 1 hour and 20 minutes 86.4 ml 4-chlorobenzoyl chloride, while the temperature was kept below 30°C. After concluding the addition of the reagent the mixture was kept at room temperature with stirring for 2.0 hours. The white solid product was filtered off and washed with water. The dry product weighed 174.0 g. Yield: 95.7% III.

### Method D

In a 2.0 liter flask were placed 77 ml water, 81.9 g sodium bicarbonate and 102.8 Tyramine base (assay - 98.1%). The stirred mixture was cooled to 15°C and 380 ml acetone was added over 15 minutes. 104.8 ml of 4-chlorobenzoyl chloride was added to the resulting mixture with cooling. Following completion of the addition the mixture was kept at room temperature for 2.0 hours and the product was filtered off. The dry product weighed 213.5 g. Yield: 96.8% III.

### Example 2

### Preparation of II from N-[2-(p-hydroxyphenyl)ethyl]-p-chloro-benzamide, III

A 2.0 liter reactor was charged with 217 ml water. The reactor was purged with nitrogen and charged with 306 g 2-ethylhexanol, 190.5 g isopropyl-2-bromoisobutyrate, 125g III, 62 g potassium carbonate anh. and 40.5 g NaOH 45% w/w aq.sol. The stirred mixture was heated to reflux for 3.5. hours. The reactor was charged with 155 ml water and reheated to 90°C. Stirrer was stopped and the reactor was kept at 90°C for 1.0 hour. The aqueous phase was separated and discarded. The reactor was charged with 155 g potassium carbonate. Nitrogen flow was discontinued. The mixture was heated over 2.5 hours to 120-130°C, then 550 ml water was added; after 0.5 hours the aqueous phase was separated and discarded. To the organic phase left in the reactor, at 60°C and fast stirring, 90 g KOH 50% weight aqueous solution was added over 4.0 hours at 40-50°C. The mixture was kept at 60°C for an additional 3.0 hours, 570 ml water was added. Stirrer was stopped; the mixture was allowed to stand at 25-30°C for 4 to 6 hours. The aqueous phase was separated and acidified to pH 4.5-5.5 with dilute sulfuric acid. The product was filtered off, washed with water and dried, to give 132.5 g II, mp 183-184°C. Yield: 81%.

### Example 3

### Preparation of II from Tyramine Hydrochloride

A 1 liter reactor, equipped with a mechanical stirrer, a condenser attached to a nitrogen trap, an equilibrated addition funnel and a thermometer, was charged with 233.0 ml water, 46.8 g sodium bicarbonate, 44.3 g sodium hydroxide 46% w/w aqueous solution, 83.3 g Tyramine hydrochloride and 200 g isopropyl bromoisobutyrate.

The stirred mixture in the reactor was purged with nitrogen. The mixture was heated to 50°C and the reactor was charged with 85 g 4-chlorobenzoyl chloride (CBC) added dropwise over 1.5 hrs. Reactor temperature was maintained in the 52-54°C range. Following completion of the CBC addition the reactor was heated to 52-54°C for 0.5 hr, then heated to reflux for 1.0 hr. The reactor was cooled to 98°C and was charged with 65.0 g potassium carbonate, 41.7 g sodium hydroxide 46% w aqueous solution and 107.5 g 2-ethylhexanol. The reactor was heated to reflux for 0.5 hr. Then stirrer was stopped and the lower aqueous layer was removed from the mixture.

The reactor, containing the warm stirred organic layer, was charged at 95°C with 100.7 g potassium carbonate. The reactor was heated over a period of 3.0 hrs from 120° to 145°C with some distillation taking place. The reactor was cooled to 120°C and was charged with 120 ml water. Stirring continued for 15 minutes, then stirrer was stopped and the mixture was allowed to separate to an aqueous (lower) layer and an organic (upper) layer. The aqueous phase was separated.

The temperature of the organic phase in the reactor was adjusted to 65°C. The reactor was purged with nitrogen and charged, over 1.5 hours, with a solution prepared from 68.2 g potassium hydroxide (90% w) and 68.2 ml water. Throughout the addition of the potassium hydroxide solution, vigorous stirring and a temperature of 65-75°C were maintained. At the completion of the addition the mixture was warmed to 75°C for and additional 1.5 hr.

The reactor was charged with 250 ml water and 40 g sulfuric acid, 40% w aqueous solution. The temperature was adjusted to 40°C, and the solution was filtered through a Celite pad. The reactor was washed with 50 ml water, the washings were filtered through the Celite pad and unified with the filtrate. The filtrate was charged into a 2 liter reactor. To the stirred filtrate warmed to 80°C, 250 ml methyl isobutyl ketone and about 50 g sulfuric acid 40% w aqueous solution were added. pH was adjusted to 4.0-4.5. Stirrer was stopped and the clear (bottom) aqueous phase was separated and discarded.

Stirrer was started. The reactor was warmed to 80°C and was charged with 300 ml water. The reactor was warmed to 90°C then cooled to 15°C. The mixture was filtered on a Buchner filter. The filter-cake was washed with 120 ml cold water, 120 ml hot water and again with 120 ml cold water and was thoroughly drained. A crude wet bezafibrate was obtained, weight 203 g.

A 1 liter reactor equipped with a mechanical stirrer, a condenser and a thermometer was charged with 420 methyl isobutyl ketone, 42 ml water and the previously isolated crude bezafibrate. The stirred reactor was heated to reflux then cooled to 15°C. The product was filtered off on a Buchner filter, the cake was washed with 120 ml portions of cold, hot, and cold water and drained. Wet weight, 204 g.

The wet product was dried at 105°C first in an oven with air circulation for 12 hours, then in a vacuum oven for 2 hours. Dry weight: 144.8 g pure bezafibrate; yield: 83.4%, based on Tyramine hydrochloride.

It will be evident to those skilled in the art that the invention is not limited to the details of the foregoing illustrative examples and that the present invention may be embodied in other specific forms without departing from the essential attributes thereof, and it is therefore desired that the present embodiments and examples be considered in all respects as illustrative and not restrictive, reference being made to the appended claims, rather than to the foregoing description, and all changes which come within the meaning and range of equivalency of the claims are therefore intended to be embraced therein.

## Claims

1. A process for the production of N-[2-(p-hydroxyphenyl) ethyl]-p-chlorobenzamide of the formula III: 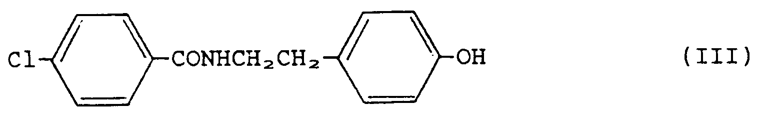 and derivatives thereof, comprising reacting 4-(2-aminoethyl) phenol (Tyramine) of formula I: 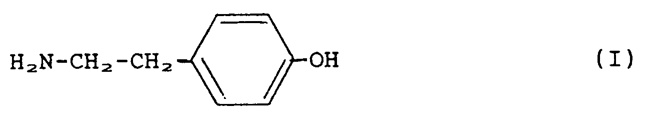 or the hydrochloride salt thereof, with 1.0 to 1.5 molar equivalent of 4-chlorobenzoyl chloride in the presence of water, in the presence of at least one molar equivalent of base and in the presence of an organic solvent.

2. A process according to claim 1, wherein said base is selected from the group consisting of potassium hydroxide, sodium hydroxide, potassium bicarbonate, potassium carbonate, pyridine, trialkylamine of formula NR₃ where R = CH3, C₂H₅, C₃H₇, C₄H₉, C₅H₁₁, or a mixture thereof.

3. A process according to claim 2, comprising reacting Tyramine hydrochloride in the presence of 1.0 to 1.1 molar equivalent of sodium or potassium hydroxide, and in the presence of 1.0 to 2.0 molar equivalents of a weak base selected from sodium bicarbonate, pyridine or trialkyl amine of formula NR₃ as defined.

4. A process according to claim 1, comprising reacting Tvramine hydrochloride with 1.0 to 1.15 molar equivalent of 4-chlorobenzoyl chloride.

5. A process according to claim 1, wherein said organic solvent is an ester, ketone or ether, or a mixture thereof.

6. A process according to claim 1, wherein said organic solvent is selected from a lower aliphatic ester of 2-bromoisobutyric acid, a lower aliphatic ester of 2-chloroisobutyric acid, or a mixture thereof.

7. A process according to claim 1, wherein said organic solvent is selected from methyl, ethyl or isopropyl 2-bromoisobutyrate, methyl, ethyl or isopropyl 2-chloroisobutyrate, or a mixture thereof.

8. A process according to claim 1, wherein said organic solvent is a lower aliphatic ester of acetic, propionic, butyric or isobutyric acid, or mixtures thereof, and the N-[2-(p-hydroxy-phenyl)ethyl]-p-chlorobenzamide produced is isolated from the reaction mixture.

9. A unitary process according to claim 1, for the production of the 2-[4-[2-[(4-chlorobenzoyl)amino]ethyl]phenoxy]-2-methyl propanoic acid derivative of N-[2-(p-hydroxyphenyl)ethyl]-p-chlorobenzamide in situ, comprising reacting Tyramine hydrochloride with 1.0 to 1.35 molar equivalent of 4-chlorobenzoyl chloride in the presence of:
a. water;
b. at least one molar equivalent of sodium or potassium hydroxide;
c. at least one molar equivalent of a weak base selected from sodium bicarbonate, potassium bicarbonate, sodium carbonate, potassium carbonate or pyridine and trialkylamine of formula NR₃ when R = CH3, C₂H₅, C₃H₇, C₄H₉, C₅H₁₁, or a mixture thereof;
d. a lower aliphatic ester of 2-bromoisobutyric acid, a lower aliphatic ester of 2-chloroisobutyric acid or a mixture thereof; and
e. a high boiling hydroxylic solvent;
to produce isopropyl 2-[4-[2(4-chlorobenzoyl)amino]ethyl]phenoxy]-2-methyl propanoate of the general formula V: 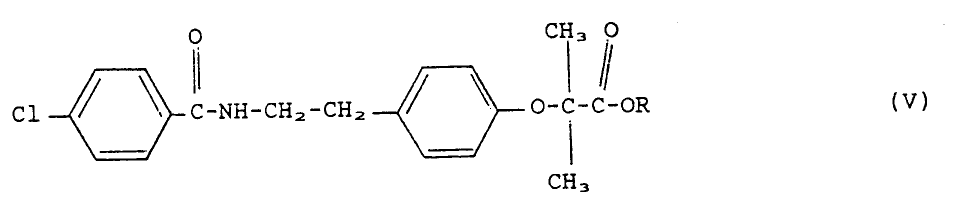 which ester is then hydrolyzed to form II through reaction with an aqueous solution of potassium hydroxide or sodium hydroxide or mixtures thereof in the temperature range of about 40°C to 90°C, preferably 65-75°C.

10. A process according to claim 9, wherein said high boiling hydroxylic solvent is a primary or secondary aliphatic alcohol.

11. A process according to claim 9, wherein the said high boiling hydroxylic solvent is one of the isomers or a mixture of isomers of octanol.

12. A process according to claim 1, for the production of the 2-[4-[2-[(4-chlorobenzoyl)amino]ethyl]phenoxy]-2-methyl propanoic acid derivative of N-[2-(p-hydroxphenyl)ethyl]-p-chlorobenzamide, wherein N-[2-(p-hydroxphenyl)ethyl]-p-chlorobenzamide is isolated and then reacted with an aqueous solution of potassium hydroxide or sodium hydroxide or mixtures thereof, to form 2-[4-[2-[(4-chlorobenzoyl)amino]ethyl]phenoxy]-2-methyl propanoic acid.

13. A process according to claim 8, for the production of the 2-[4-[2-[(4-chlorobenzoyl)amino]ethyl]phenoxy]-2-methyl propanoic acid derivative of N-[2-(p-hydroxphenyl)ethyl]-p-chlorobenzamide, wherein N-[2-(p-hydroxphenyl)ethyl]-p-chlorobenzamide is isolated and then reacted with a lower aliphatic ester of 2-bromo or 2-chloroisobutyric acid or mixtures thereof, to form a lower aliphatic ester of 2-4-[2-[(4-chlorobenzoyl)amino]ethyl]phenoxy]-2-methyl propanoic acid which is hydrolyzed to II through reaction with an aqueous solution of potassium hydroxide or sodium hydroxide or a mixture thereof, in the temperature range of 40-90°C.

14. A process according to claim 1, comprising introducing methyl isobutyl ketone or a mixture of methyl isobutyl ketone and water to effect extractive purification and recrystallization of 2-[4-[2-[(4-chlorobenzoyl)amino]ethyl]phenoxy]-2-methyl propanoic acid.

15. N-[2-(p-hydroxyphenyl)ethyl]-p-chlorobenzamide (III) prepared according to claims 1-8.

16. 2-[4-[2-[(4-chlorobenzoyl)amino]ethyl]phenoxy]-2-methyl propanoic acid (II) prepared according to claims 9-14.
